(19) Europäisches Patentamt
European Patent Office
Office européen des brevets

(11) **EP 2 036 490 B1**

(12) **EUROPEAN PATENT SPECIFICATION**

(45) Date of publication and mention
of the grant of the patent:
**25.02.2015   Bulletin 2015/09**

(51) Int Cl.:
*A61B 5/00* (2006.01)          *A61B 8/00* (2006.01)

(21) Application number: **08164180.5**

(22) Date of filing: **11.09.2008**

(54) **Measurement apparatus**

Messvorrichtung

Appareil de mesure

(84) Designated Contracting States:
**DE FR IT**

(30) Priority:   **12.09.2007   JP 2007237320**

(43) Date of publication of application:
**18.03.2009   Bulletin 2009/12**

(73) Proprietor: **Canon Kabushiki Kaisha
Tokyo 146-8501 (JP)**

(72) Inventor: **Nishihara, Hiroshi
Ohta-ku Tokyo (JP)**

(74) Representative: **Hitching, Peter Matthew et al
Canon Europe Ltd
3 The Square
Stockley Park
Uxbridge
Middlesex
UB11 1ET (GB)**

(56) References cited:
WO-A-2005/057561          WO-A-2006/079013
US-A- 6 002 958          US-A1- 2005 256 403
US-A1- 2006 058 685          US-A1- 2007 187 632

• ZEMP R ET AL: "Stochastic explanation of
speckle contrast detection in ultrasound-
modulated optical tomography" PHYSICAL
REVIEW E (STATISTICAL, NONLINEAR, AND
SOFT MATTER PHYSICS) APS THROUGH AIP
USA, vol. 73, no. 6, June 2006 (2006-06), pages
61920-1, XP002507999 ISSN: 1539-3755

**Description**

BACKGROUND OF THE INVENTION

Field of the Invention

The present invention relates to a measurement apparatus configured to measure a spectroscopic characteristic of a specimen by Acousto-Optical Tomography.

Description of the Related Art

Conventional measurement apparatuses such as mammography apparatus can measure a spectroscopic characteristic in a biological tissue. Some of these conventional spectroscopic measurement apparatuses apply Acousto-Optical Tomography ("AOT"). In AOT, coherent light is irradiated onto and ultrasound is focused into the biological tissue, and the modulated light is detected by a light detecting unit using an effect of light modulation (acousto-optical effect) in a ultrasound focused area, as is described in U.S. Patent No.6,738,653.

The light signal intensity to be detected as a result of the light modulation effect by the ultrasound varies with the size of an area (a surface area) on which the ultrasound and the light interact with each other. See, for example, Lihong V. Wang, "Ultrasonic Modulation of Scattered Light in Turbid Media and a Potential Novel Tomography in Biomedicine," Photochemistry and Photobiology, 1998, 67 (1) : 41-49. The light modulation depth also varies with the ultrasound intensity in the area where the ultrasound and the light interact with each other. See, for example, Lihong V. Wang, "Mechanism of Ultrasonic modulation of Multiply Scattered Coherent Light: An Analytical Model," Phys. Rev. Lett., vol.87, No. 4, 2001. The light modulation depth further varies with the frequency of the ultrasound in an area where the ultrasound and the light interact with each other. See, for example, Lihong V. Wang, "Ultrasonic modulation of multiply scattered coherent light: An analytical model for anistropically scattering media," PHYSICAL REVIEW E 66, 026603, 2002.

A smaller ultrasound focusing size on a measurement site improves the resolution on the measurement site on the AOT. On the other hand, a larger ultrasound focusing size onto the measurement site improves (increases) the modulation depth for the modulated light as a detection signal, and facilitates a detection of the modulated light. In scanning a measurement site (an ultrasound focusing position) in order to measure an internal tissue in a specimen, a measurement time period shortens as the ultrasound focusing size on the measurement site becomes larger.

The conventional AOT measurement apparatuses disclosed in U.S. Patent No. 6, 738, 653 and "Ultrasonic Modulation of Scattered Light in Turbid Media and a Potential Novel Tomography in Biomedicine" set a constant focusing size on the measurement site. Thus, they have a fixed resolution, a fixed modulation depth, and a fixed measurement time, and cannot meet a demand for adjustment. For example, if a poor SN ratio causes a measurement failure and a worthless measurement result, there is a demand for an improved SN ratio to obtain a measurement result by the smallest sacrifice of the resolution in this case. High speed scanning and a shortened measurement time period with a degraded resolution are demanded in areas with no abnormality. On the other hand, a thorough measurement with an improved resolution is demanded in an area that is abnormal or possibly abnormal (hereinafter referred as "abnormal") irrespective of a measurement time period.

SUMMARY OF THE INVENTION

The present invention provides a measurement apparatus which can flexibly meet such user demands.

The present invention in its first aspect provides a measurement apparatus as specified in claims 1 to 9. Document US 6 002 958 discloses an apparatus and method according to the preambles of claims 1 and 9, respectively.

Further features of the present invention will become apparent from the following description of exemplary embodiments (with reference to the attached drawings).

BRIEF DESCRIPTION OF THE DRAWINGS

FIG. 1 is a block diagram of a measurement apparatus according to a first embodiment of the present invention.

FIG. 2 is a schematic perspective view of an ultrasound generating unit on the measurement apparatus shown in FIG. 1.

FIG. 3 is a sectional view of the ultrasound generating unit taken along a line A in FIG. 2.

FIG. 4 is a block diagram of an ultrasound focusing unit of the measurement apparatus shown in FIG. 1.

FIG. 5 is another block diagram of the ultrasound focusing unit of the measurement apparatus shown in FIG. 1.

FIG. 6 is yet another block diagram of the ultrasound focusing unit in the measurement apparatus shown in FIG. 1.

FIG. 7 is a graph showing a pressure distribution at an ultrasound focusing position shown in FIG. 6.

FIG. 8 is a graph showing a shot noise characteristic of an optical sensor in a light detecting unit shown in FIG. 1.

FIG. 9 is a flow chart which describes how the measurement apparatus shown in FIG. 1 operates.

FIG. 10 is a block diagram of a measurement apparatus according to a second embodiment of the present invention.

FIG. 11 is a flow chart which describes how the measurement apparatus in FIG. 10 operates.

FIG. 12 is a graph which shows absorption spectra of $HbO_2$ and Hb in wavelengths between 600 and 1000nm.

## DESCRIPTION OF THE EMBODIMENTS

Referring now to the accompanying drawings, a description will be given of embodiments of the present invention.

## FIRST EMBODIMENT

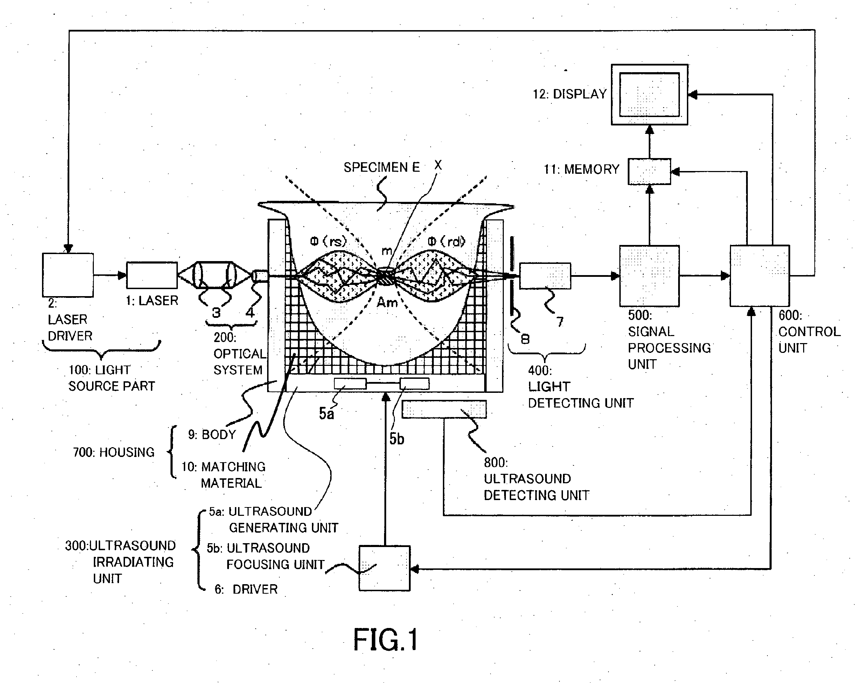

FIG. 1 is a block diagram of a measurement apparatus according to a first embodiment of the present invention. The measurement apparatus uses AOT to measure a spectroscopic characteristic of a measurement site X (or a focusingposition) in an internal tissue of a specimen E. The measurement apparatus includes a light source part 100, an optical system 200, an ultrasound irradiating unit 300, a light detecting unit 400, a signal analyzing (processing) unit 500, a control unit 600, a housing 700, and an ultrasound detecting unit 800.

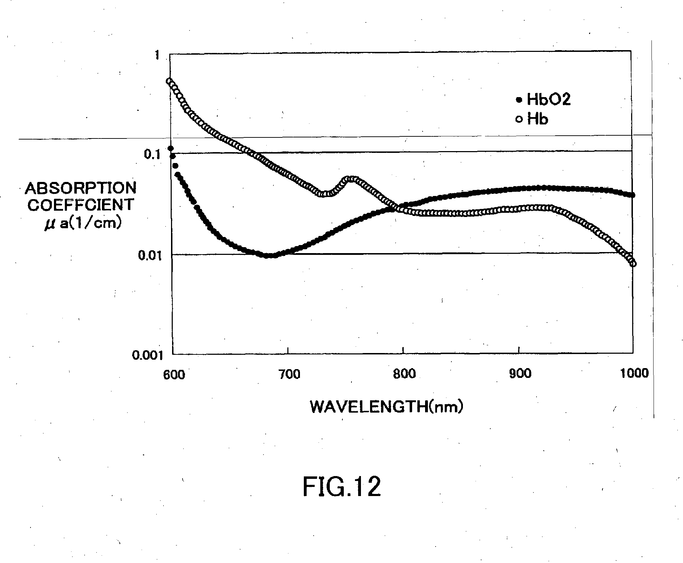

The specimen E is a biological tissue, such as a breast. It is known that a new blood vessel starts to form or that oxygen consumption increases as a tumor such as a cancer grows. Absorption spectroscopic characteristics of oxygenated hemoglobin ($HbO_2$) and reduced hemoglobin (Hb) may be used to evaluate formation of the new blood vessel or an increase in the oxygen consumption. FIG. 12 shows absorption spectra of $HbO_2$ and Hb in wavelengths between 600 and 1000nm.

The measurement apparatus measures Hb and $HbO_2$ concentrations in blood in a biological tissue from absorption spectra of $HbO_2$ and Hb at a plurality of wavelengths, measures the Hb and $HbO_2$ concentrations at multiple positions, and generates images of concentration distributions. Thus areas where new blood vessels are formed can be identified in the biological tissue. The measurement apparatus calculates oxygen saturation from the Hb and $HbO_2$ concentrations, and enables an area where the oxygen consumption increases to be identified by the oxygen saturation. The spectroscopic information of Hb and $HbO_2$ measured by the measuring apparatus can be used for diagnostics.

The light source part 100 includes a laser 1 as a light source and a laser driver 2, and emits luminous fluxes having a plurality of wavelengths. For example, the laser 1 has a long coherence length, such as 1 m or greater, and generates continuous wave ("CW") light having a constant intensity. The laser 1 of this embodiment is configured to change the intensity under control by the control unit 600. A wavelength $\lambda 0$ is selected among wavelengths in accordance with absorption spectra such as water, lipid, protein, oxygenated hemoglobin, and reduced hemoglobin. In an example, an appropriate wavelength falls in a range between 600 to 1500 nm, because the light can be highly transmitted due to the small absorption by water that is a main ingredient of the internal biological tissue, and the spectra of the lipid, the oxygenated hemoglobin, and the reduced hemoglobin are characteristic. The laser 1 may use a semiconductor laser or a wavelength-variable laser which generate various different wavelengths.

The optical system 200 includes a lens group 3 and an optical fiber 4, and guides the light from the light source part 100 to the specimen E. The lens group 3 is a condenser optical system configured to efficiently guide the light from the laser 1 to an edge of the optical fiber 4. The optical fiber 4 is a light guiding system configured to guide the light to the specimen E housed in housing 700.

The ultrasound irradiating unit 300 includes an ultrasound generating unit 5a, an ultrasound focusing device 5b, and a driver 6. The driver 6 drives the ultrasound generating unit 5a and the ultrasound focusing unit 5b.

The ultrasound generating unit 5a generates ultrasound (or an ultrasonic pulse). The ultrasound generating unit 5a is configured to adjust an ultrasonic intensity and/or frequency under control of the control unit 600. This embodiment sets an ultrasonic frequency $\Omega 0$ in a range between 1 and several tens of MHz, although an appropriate frequency depends on the required measurement depth or resolution of the specimen E.

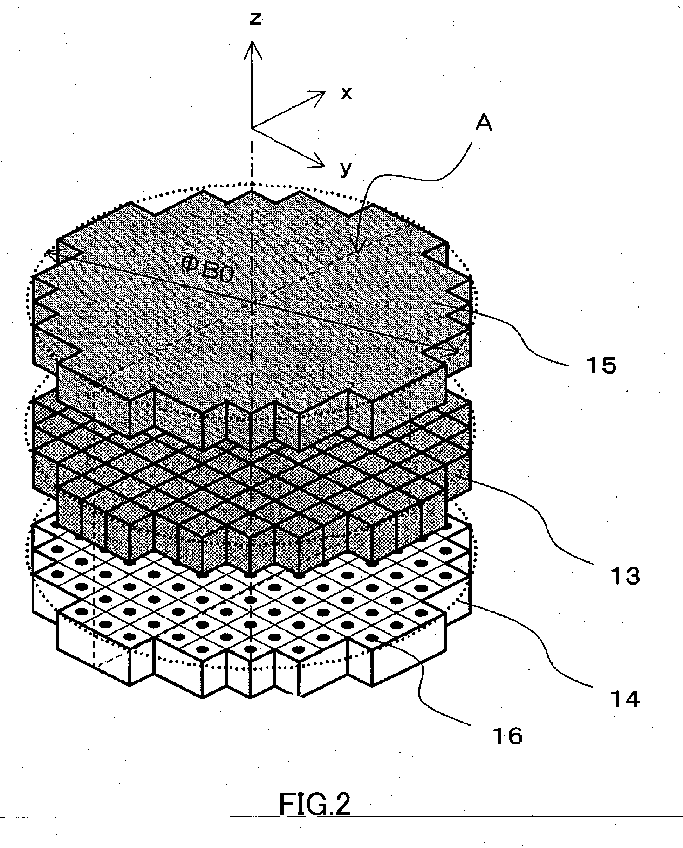

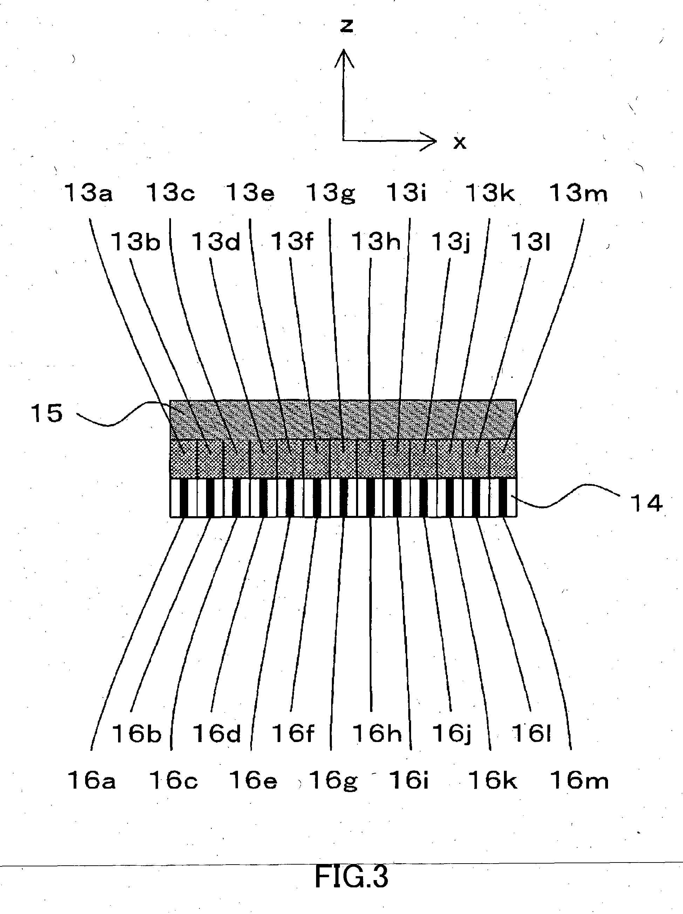

FIG. 2 is a schematic perspective view of a structure of a 2D array search unit as an example of the ultrasound generating unit 5a. FIG. 3 is a sectional view of a line A in FIG. 2. A plurality of small square-rod shaped ultrasonic transducers 13 are arranged on a plurality of backing members 14 within an area having a diameter B0. An acoustic matching layer 15 is arranged on an ultrasound irradiating surface of the ultrasonic transducer 13. A lead wire 16 is connected to each ultrasonic transducer 13.

The ultrasonic transducer 13 includes piezoelectric elements, each of which provides a piezoelectric effect which converts an applied voltage into ultrasound, or converts a received pressure change into a voltage. The piezoelectric element may use a piezoelectric ceramic material as typified by lead zirconate titanate ("PZT") or a polymer piezoelectric membrane material as typified by polyvinylidene-fluoride ("PVDF"). A device which converts an ultrasonic mechanical oscillation into an electric signal or an electric signal into an ultrasonic mechanical oscillation is referred to as an ultrasonic transducer.

The backing member 14 absorbs an acoustic wave that propagates in a direction opposite to a traveling direction of the ultrasound, and restrains unnecessary oscillations of the ultrasound transducer 13. Since a piezoelectric element is significantly different from a biological body in acoustic impedance, a direct contact between the piezoelectric element and the biological tissue causes a reflection on the interface to be too large to efficiently transmit the ultrasound. For this reason, the acoustic matching layer 15 made of a material having an intermediate acoustic impedance is inserted between the ultrasound transducer 13 composed of the piezoelectric elements and the biological body, to efficiently transmit the ultrasound.

The lead wire 16 transfers a driving signal voltage from the driver 6 to the ultrasonic oscillator 13.

The ultrasound focusing unit 5b focuses the ultrasound from the ultrasound generating unit 5a onto the measurement site X in the specimen E. The ultrasound focusing unit 5b is configured to change a focusing size under control by the control unit 600. Methods of focusing the ultrasound may include using a concave ultrasonic transducer or an acoustic lens that has a spherical, a cylindrical, or an aspheric shape, or electronic focusing that utilizes an array search unit. On the concave ultrasonic transducer, a curvature on the concave surface determines the focusing position, and the focal length and diameter of the transducer determine the focusing size. The acoustic lens has a convex shape if made of a material having a sonic speed lower than that in the biological tissue. Like the concave ultrasonic transducer, the curvature of the convex surface determines the focusing position, and the focal length and diameter of an acoustic lens determine the focusing size.

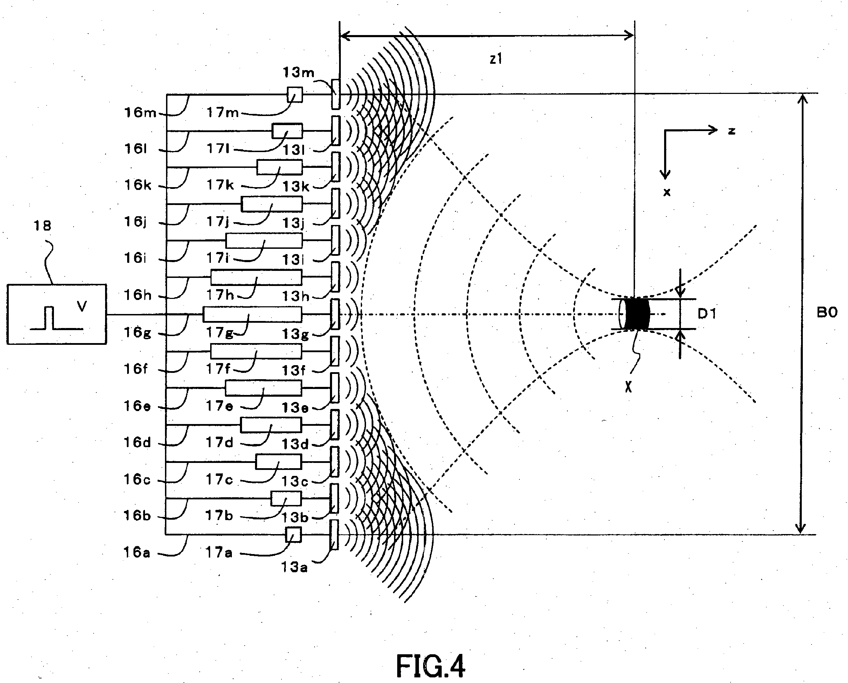

This embodiment applies electronic focusing that uses a 2D array search unit described above. Referring now to FIG. 4, a description will be given of an illustrative ultrasound focusing unit 5b. Here, FIG. 4 is a block diagram as an example of the ultrasound focusing unit 5b. This embodiment will describe only a relationship in an X direction for convenience, although this description is also true of the y direction.

Variable delay elements 17a to 17m and a pulse generator 18 are respectively connected to a plurality of the arranged ultrasonic transducers 13a to 13m via the lead wires 16a to 16m. The variable delay element 17 uses a coil-shaped thin electric wire to delay transmission of an electric signal. A delay time period of the electronic signal is adjustable by switching a plurality of taps which are provided on the coil. The pulse generator 18 is a device that generates a pulse voltage applied to the ultrasonic transducer 13.

Assume that $\tau a$ to $\tau m$ are delay time periods given to the ultrasonic transducers 13a to 13m. When a longer delay time period is set to a variable delay element 17 that is closer to the center (e.g., $\tau a = \tau m < \tau b = \tau l < \tau c = \tau k < \tau d = \tau j < \tau e = \tau i < \tau f = \tau h < \tau g$), as shown in FIG. 4, a synthesis wavefront formed by each ultrasonic transducer 13 becomes a focused wavefront.

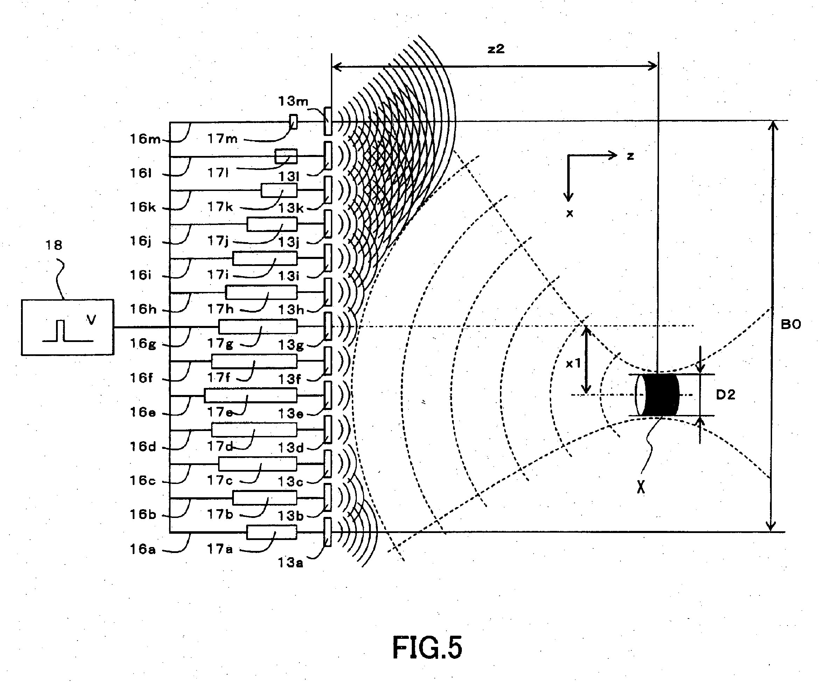

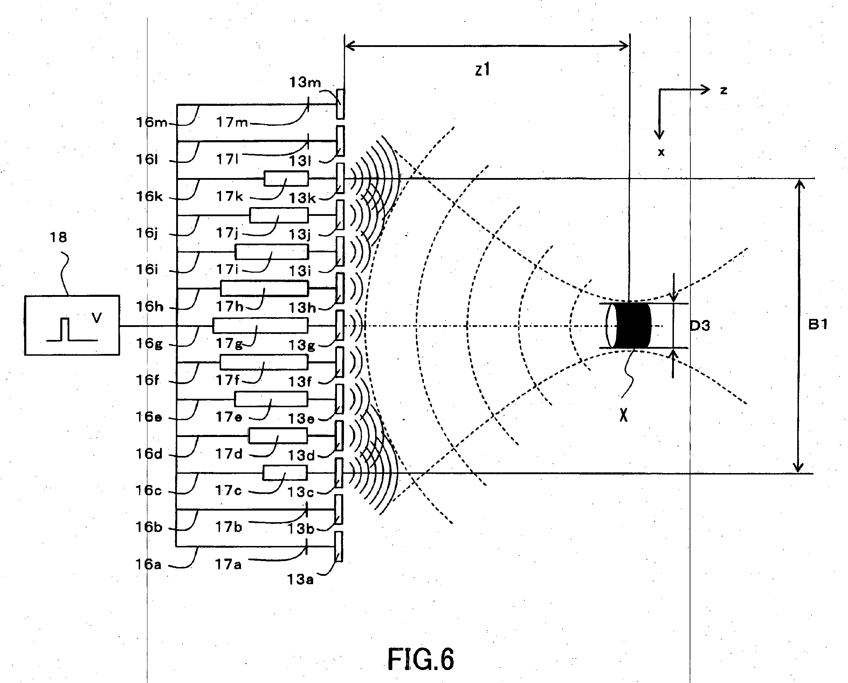

In FIG. 4, all the ultrasonic transducers 13 are driven (in a set range B0) and the ultrasound is condensed from the central transducer 13g in the z direction by a horizontal distance z1 onto a measurement site (focusing position) X having a condensing diameter D1. In FIG. 5, all the ultrasonic transducers 13 (in the set range B0) are driven, and the ultrasound is condensed from the central transducer 13g in the z direction by a horizontal distance z2 and in the x direction by a horizontal distance x1 onto a measurement site (focusing position) X having a condensing diameter D2. In FIG. 6, part of the ultrasonic transducers 13 is driven (in a set range B1), and the ultrasound is condensed from the central transducer 13g in the z direction by a horizontal distance z1 onto a measurement site (focusing position) X having a condensing diameter D3. FIG. 6 changes the focusing diameter D1 in FIG. 4 to the focusing diameter D3. Thus, control over a range of the transducers 13 and a delay time period given by the variable delay element 17 can provide control over a focusing position X, a focusing diameter and a traveling direction of the ultrasound.

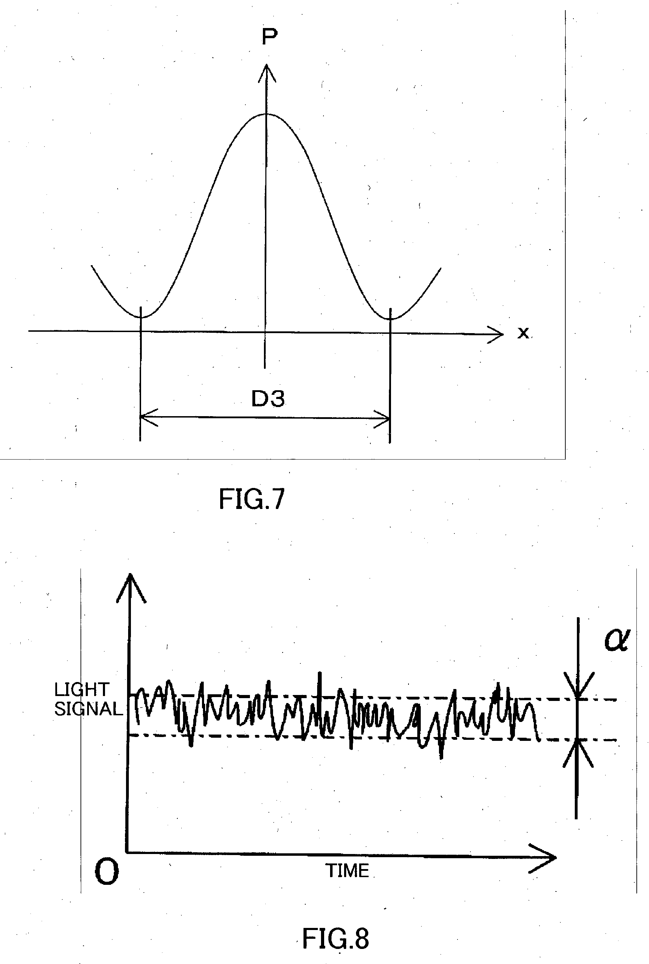

FIG. 7 is a graph of a distribution of a pressure P in the x direction at the focusing position of the ultrasound shown in FIG. 6. The focusing diameter D3 is given approximately by the following equation:

$$\text{Equation 1}$$

$$D3 = 2.44 \cdot \frac{vs}{\Omega} \cdot \frac{z1}{B1}$$

where vs is a velocity of the ultrasound that propagates in the specimen E.

Electronic focusing search units other than the 2D array search unit include a linear array search unit, which linearly arranges ultrasonic transducers, and an annular array search unit, which arranges transducers in concentric ring shapes. In using a concave ultrasonic transducer having a spherical, cylindrical, or aspheric shape, or an acoustic lens, the focusing position of the ultrasound can be controlled by mechanically driving and changing the positions of those members.

The light detecting unit 400 detects the modulated light which has been modulated as a result of the acousto-optical effect on the measurement site X in the specimen E. The light detecting unit includes a light sensor 7 and an aperture 8. The light sensor 7 may have a photoelectric conversion element, such as a photomultiplier ("PMT"), a charge coupled device ("CCD"), or a complementary metallic oxidized film semiconductor ("CMOS") device. However, the selected light sensor needs to have a sufficient sensitivity to the light having a wavelength $\lambda 0$ (such as in a range between 600 and 1500 nm) generated by the light source part 100. The aperture 8 has an opening which allows the light that propagates in the tissue of the specimen E and exits to the outside of the housing 700 to pass through it, and a shielding member which blocks the light. The aperture 8 serves to limit the amount of light guided to the light sensor 7.

The light sensor 7 has a shot noise characteristic shown in FIG. 8, and cannot detect the light unless the detected light is larger than the shot noise $\alpha$. The detected light may be twice to ten times as high as the

shot noise $\alpha$, for example. A permissible incident light intensity $\beta$ is set to the light sensor 7, and the light sensor 7 cannot receive light having an intensity exceeding $\beta$. The shot noise $\alpha$ corresponds to a value obtained at the permissible incident light intensity $\beta$, and the shot noise becomes larger than $\alpha$ if the light intensity is smaller than $\beta$. A bandpass filter or a lock-in amplifier may be used to "efficiently detect the light at a noise level smaller than the shot noise level $\alpha$ in the light sensor 7."

The light incident upon the housing 700 from the optical fiber 4 shown in FIG. 1 repeats absorptions and scatterings in the matching material 10 and the specimen E several times, and then propagates in various directions. The propagation of the light in the absorption-scattering medium may be described by a light diffusion equation, where $\varphi$ (rs) is a fluence rate of a photon derived from the light' s propagation from the laser 1 to the focused ultrasound, and $\varphi$ (rd) is a fluence rate of a photon derived from the light' s propagation from the focused ultrasound to the light sensor 7.

The acoustic pressure increases near the ultrasound's focusing position X, changes the density and the refractive index in the absorption-scattering medium, and displaces the absorption-scattering medium. When the light passes through the area on which the ultrasound is focused, the optical phase of the light changes due to a change of the refractive index and a displacement of the absorption-scattering medium. The acoustic pressure locally increases at the focusing position X, and the focusing position X is more strongly affected by the ultrasound than the peripheral part. Thus, a larger amount of the modulated light that is modulated by the ultrasound with a frequency $\Omega$ (MHz) is generated at the position X than in its peripheral areas. The spectroscopic characteristic in the measurement site X may be measured by selectively detecting the modulated light caused by the acousto-optical effect.

"Ultrasonic Modulation of Scattered Light in Turbid Media and a Potential Novel Tomography in Biomedicine" cited previously, discloses that the intensity of the modulated light caused by the asousto-optical effect depends upon the focusing size (the surface area) at the focusing position X. Assume that m is a modulation depth by which the light is modulated by ultrasound having a permissible ultrasound intensity $\gamma$ . The permissible ultrasound intensity $\gamma$ is an intensity of the ultrasound that is permitted for irradiation onto a biological tissue, and the Food and Drug Administration ("FDA") defines an upper limit of the permissible ultrasound intensity $\gamma$ to be 720 mW/cm$^2$. A light signal Iac to be detected is given by the following equation, where Am is a surface area in an interacting area (the surface area of the ultrasound focusing area), and I0 is an intensity of the incident light. Am depends on the focusing size of the ultrasound, and the light signal Iac can be set to an appropriate intensity by controlling the focusing size. It is understood from Equation 2 that the light signal Iac increases as the intensity I0 of the incident light increases. The following safety standard defies a maximum permissible exposure $\delta$ ("MPE") of the intensity of the light which is permitted to be irradiated onto the biological tissues (IEC 60825-1: Safety of laser products, JIS C 6802: Safety of laser products, FDA: 21CFR Part 1040. 10, ANSI Z136.1: Laser Safety Standards, etc) . The maximum permissible exposure $\delta$ is set depending upon a wavelength of an irradiated light or an exposure time period, and the light intensity can be varied as long as it does not exceed $\delta$.

$$\text{Equation 2}$$
$$\text{Iac} = \text{I0} \cdot \Phi(\text{rs}) \cdot \text{m} \cdot \text{Am} \cdot \Phi(\text{rd})$$

"Mechanism of Ultrasonic modulation of Multiply Scattered Coherent Light: An Analytical Model, " cited previously discloses that the modulation depth m changes as the ultrasonic intensity changes. This means that the light signal Iac can be set to an appropriate intensity by changing the ultrasonic intensity in a range that does not exceed the permissible ultrasonic intensity of 720 mW/cm$^2$ defined by the Food and Drug Administration ("FDA").

"Ultrasonic Modulation of Scattered Light in Turbid Media and a Potential Novel Tomography in Biomedicine" cited previously further discloses that a numerical value related to the modulation depth m, which is 1-$G_1(0.5T_a)$ in its description, changes when the frequency of the ultrasound changes. This reference indicates that the modulation depth increases as the frequency of the ultrasound increases. On the other hand, it is understood from Equation 1 that the focusing size reduces as the ultrasonic frequency $\Omega$ increases. Equation 1 also indicates that the focusing size changes in the ultrasonic transducer' driving range B1. At this time, for example, a combination of B1 and $\Omega$ may be selected which increases the ultrasound frequency without changing the focusing size. In other words, the light signal Iac can be properly set by changing the ultrasonic intensity.

The light sensor 7 detects both the modulated light modulated by the ultrasound, and the multi-scattered, non-modulated light that is free of ultrasonic modulation.

The light sensor 7 can measure a light signal at a desired position by controlling (or scanning) the ultrasound focusing position X through the ultrasound irradiating unit 300.

The ultrasound detecting unit 800 detects the intensity of the ultrasound which is focused in the specimen E. Based on a detection result of the ultrasound detecting unit 800, the control unit 600 controls the intensity of the ultrasound generated by the ultrasound unit 5a. The control unit 800 includes a piezoelectric device like the ultrasound generating unit 5a, and thus can be one device (ultrasonic transducer) that has both transmitting and receiving functions in an example of the above search unit.

The signal analyzing unit 500 analyzes the output of the light detecting unit 400, and informs the control unit 600 of the result of the analysis. The control unit 600 may perform a part or all of the signal analyzing functions, as an alternative. In the latter case, the signal analyzing unit 500 and the control unit 600 are integrated.

The signal analyzing unit 500 of this embodiment first transmits intensity information of the light signal which mixes the non-modulated light with the modulated light caused by the ultrasound having the frequency $\Omega$ (MHz) detected by the light sensor 7 in the light detecting unit 400 to the control unit 600. The signal analyzing unit 500 also extracts the light signal Iac of the modulated light from the light signal that mixes the non-modulated light with the modulated light caused by the ultrasound detected by the light sensor 7. The signal analyzing unit 500 may extract or separate the modulated light from the non-modulated light using a filter (not shown). The filter may be a band pass filter which selectively detects a signal having a specific frequency, or a lock-in amplifier which detects by amplifying the light of a specific frequency. The signal analyzing unit 500 also compares the level of the optical signal Iac of the modulated light with the level in the shot noise $\alpha$ in the light sensor 7 (which is a threshold twice as high as the noise level in this embodiment.) and informs the control unit 600 of the result of the comparison. The threshold is stored in a memory 11 which will be described later. The signal analyzing unit 500 produces a distribution of a spectroscopic characteristic in the specimen based on coordinate data of the focusing position X and the light signal Iac corresponding to the coordinate data.

Based on an output of the light detecting unit 400, the control unit 600 controls at least one of an ultrasonic intensity or frequency the ultrasound generating unit 5a, an ultrasonic focusing size by the ultrasound focusing unit 5b. In this embodiment, an output of the light detecting unit 400 is obtained as a comparison result with the signal detecting unit 500. The control unit 600 also controls each unit in the measurement apparatus. The control unit 600 serves as a signal processing device with the signal analyzing unit 500, and generates an image of the spectroscopic characteristic of the measurement site in the specimen E. The control unit 600 has an image generating function, and generates an image from distribution data of the spectroscopic characteristic in the specimen, which the signal analyzing unit 500 generates.

The memory (storage means) 11 stores an operation flow of the measurement apparatus which will bedescribedhereinafter, data used for the apparatus (such as the level of the shot noise $\alpha$), and an image of the spectroscopic characteristic generated by the measurement apparatus. The memory 11 can use a data storage means such as an optical disk, a magnetic disk, a semiconductor memory, or a hard disk drive. The display 12 displays an image that the measurement apparatus generates, and can use a display device such as a liquid crystal display, a CRT, and an organic EL.

The housing 700 consists of a body 9 filled with the matching material 10, and houses the specimen E. The body 9 serves as a vessel which houses the specimen E and the matching material 10. The body 9 is made of a material which transmits the light having a wavelength $\lambda0$ (for example, in a range between 600 and 500 nm) generated by the light source part 100. The matching material 10 is an acoustic impedance material which efficiently transmits the ultrasound from the ultrasound irradiating unit 300 to the specimen E.

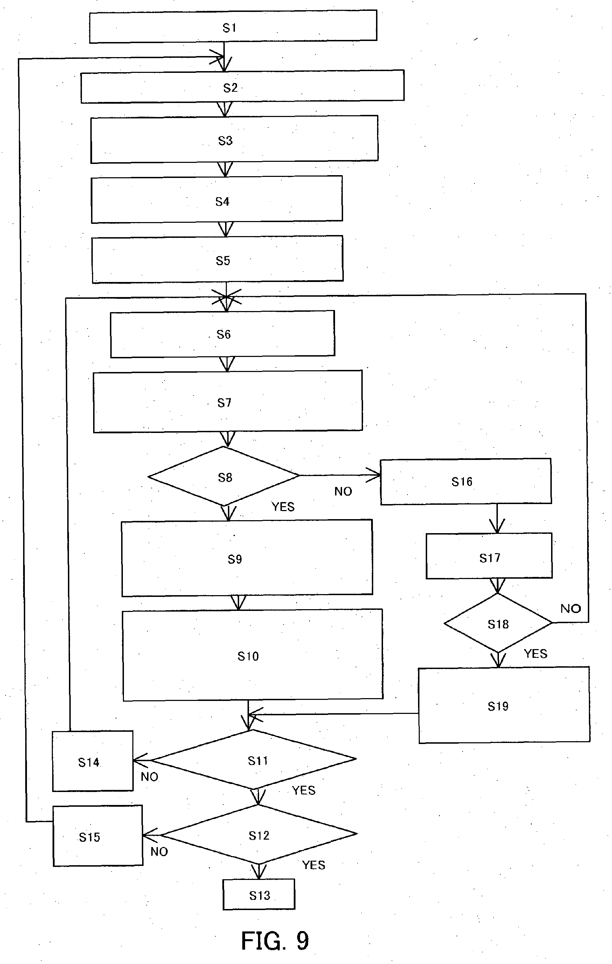

Referring now to FIG. 9, a description will be given of an operation of the measurement apparatus. FIG. 9 is a flowchart that describes how the measurement apparatus operates. S means a step in FIG. 9.

First, the control unit 600 sets an initial position r0 of the measurement site in step S1.

Next, the control unit 600 sets a wavelength of the light in the light source part 100 to an initial value $\lambda1$ in step S2. This embodiment uses three types of wavelengths $\lambda1$, $\lambda2$, and $\lambda3$ as appropriate wavelengths of the light in order to obtain spectroscopic characteristics of oxygenated hemoglobin (HbO$_2$) and reduced hemoglobin (Hb). These wavelengths are selected based on the characteristic of the spectrum shown in FIG. 12, for example, as 800 nm where the absorption characteristics of HbO$_2$ and Hb reverse, and 720 nm and 970 nm before and after 800 nm where a difference in the absorption characteristic becomes large ($\lambda1 = 720$ nm, $\lambda2 = 800$ nm, $\lambda3 = 970$ nm).

Next, the control unit 600 sets a focusing size of the ultrasound focusing unit 5b to an initial value D1 in step S3. In this embodiment, a maximum focusing size to be set is 10 mm, at which, for example, a breast cancer tumor is likely to grow drastically. D1 is set in this range.

Next, the control unit 600 sets an intensity of the ultrasound generated by the generating unit 5a to an initial value F1 in step S4. First, the ultrasound generating unit 5a is set to an initial value F1 having the frequency $\Omega$ (MHz) and an intensity that does not exceed the permissible ultrasonic intensity $\gamma$. Next, the control unit 600 controls the intensity of theultrasoundgenerated by the ultrasound generating unit 5a so that intensity actually detected by the ultrasound detecting unit 800 can be the initial value F1. As a result, the intensity detected by the ultrasound detecting unit 800 becomes the initial value F1. The control unit 600 also sets a frequency of the ultrasound from the ultrasound generating unit 5a to an initial value $\Omega1$ in step S5.

Next, the control unit 600 sets an intensity of the light generated by the light source part 100 to an initial value G1 in step S 6. Initially, the light source part 100 sets the continuous light to have an intensity G1 that does not exceed the permissible incident light intensity $\beta$ and the maximum permissible exposure $\delta$ in the light sensor 7. Next, the optical system 200 introduces the light to the specimen E, and the signal analyzing unit 500 transmits to the controller 600 intensity information of the light signal that mixes the non-modulated light with the modulated light detected by the light detecting unit 400. Next, the control unit 600 controls the intensity of the light generated by the light source part 100 such that the mixed light signal actually becomes G1. As a result, the intensity detected by the light detecting unit 400 becomes G1.

The measurement starts as soon as the initial value is adjusted. As a result, the continuous light having the wavelength $\lambda 1$ and the intensity of G1 is emitted from the light source part 100 and irradiated onto the specimen E via the optical system 200, and the ultrasound having the intensity F1 is focused with the focusing size D1 onto the initial position r0 by the ultrasound irradiating unit 300. Next, the signal analyzing unit 500 extracts the light signal Iac generated by modulated light from the light signal which mixes the non-modulated light with the modulated light detected by the light detecting unit 400 in step S7. Next, the signal analyzing unit 500 determines whether (an absolute value of) a level of the light signal is greater than a value that is twice as high as the level of the shot noise $\alpha$ (or Iac > $2\alpha$) in step S8. The signal analyzing unit 500 informs the control unit 600 of a comparison result between the level of the light signal Iac and the value that is twice as high as the level of the shot noise $\alpha$ or a determination result of Iac > $2\alpha$. In the formula Iac > $2\alpha$, Iac approximately represents the level of the light signal and $2\alpha$ approximately represents a value that is twice as high as the noise level. The present invention is not necessarily limited to the value that is twice as high as the noise level of the shot noise $\alpha$, as described above.

In case of Iac > $2\alpha$ (or when S8 answers yes), the signal analyzing unit 500 produces distribution data of the spectroscopic characteristic in the specimen E based on the coordinate data of the focused ultrasound and the light signal Iac that corresponds to the coordinate data in step S9. The control unit 600 records in the memory 11 the coordinate data of the focused ultrasound, the light signal Iac that corresponds to the coordinate data, and the distribution data of the spectroscopic characteristic in the specimen E which the signal analyzing unit 500 generates in step S10.

Next, the control unit 600 determines whether a set position has been measured with all wavelengths in step S11. If the set position has been measured with all wavelengths in S11, it is determined whether the entire specimen E has been measured in step S12. If the entire specimen E has been measured in S12, the control unit 600 completes the measurement, reconstructs 3D distribution data based on data at each measurement site recorded in the memory 11, and displays the data on the display 12 in step S13.

On the other hand, if the set position has not been measured with all wavelengths in S11, the control unit 600 sets a new wavelength of the light at the light source part 100 in step S14 and returns to step S6. If the entire specimen E has not been measured in S12, the control unit 600 sets a new measurement area (a focusing position) in step S15 and returns to step S2. The measurement area may be distributed either continuously or discretely in the specimen E.

When they are lower than the level of the optical signal Iac and the level of the shot noise $\alpha$ or when the answer ion step S8 is NO, the control unit 600 increases at least one of the ultrasonic intensity, the ultrasonic frequency, and the focusing size in step S16. More specifically, the control unit 600 sets to a new value G1 of the light intensity from the light source part 100a a value (G1+g) that is made by adding an increment value g to the initial value G1. The control unit 600 may set to a new value F1 of the ultrasonic intensity in the ultrasound generating unit 5a a value (F1+f) that is made by adding an increment value f to the initial value F1. The control unit 600 may also set to a new value $\Omega 1$ of the ultrasonic frequency in the ultrasound generating unit 5a a value ($\Omega 1+\omega$) that is made by adding an increment value $\omega$ to the initial value $\Omega 1$. The control unit 600 may set to a new value D1 of the focusing size by the ultrasound focusing unit 5b a value (D1+d) that is made by adding an increment value d to the initial value D1. These steps maybe combined, and the flow returns to step S7. Each increment value is preset and stored in the memory 11.

In changing the focusing size, the control unit 600 determines the focusing size by using Equations 1 and 2. When the ultrasound focusing size increase, the ultrasonic intensity changes or decreases at the focusing position. Accordingly, the intensities of the ultrasound or the incident light may also be increased where necessary. In addition, when the frequency of the ultrasound increases, the focusing size reduces, and thus the focusing size or the intensity of the incident light may also be increased where necessary. Of course, the ultrasonic intensities of the ultrasound or the incident light need to increase but keep below the upper limits of $\beta$, $\gamma$ and $\delta$.

Afterwards, the flow returns to step S7 but, in order to prevent an endless loop, the control unit 600 increments an internal counter value C at each change in step S17, and determines if the number of repetitions reaches the permissible number of times t in step S18. If the number of repetitions does not reach the permissible number of times t (when step S18 answers NO), the flow returns to step S6. If the internal counter value C reaches the permissible number of times t (when step S18 answers YES), the control unit 600 indicates an error message on the display 12 and stores the coordinate data, the wavelength, and the non-measurable state information in the memory 11 in step S19. After S19, the flow moves to step S11.

In this embodiment, the control unit 600 scans the focusing site X of the ultrasound using the ultrasound focusing unit 5b (to change the measurement area). When a SN (signal to noise) ratio of the modulated light at the first ultrasound focusing position is below the threshold (when the signal level of the modulated light is below the level of the shot noise $\alpha$), one of the intensity and the frequency of the ultrasound, the focusing size may be varied or increased. Then, a measurement is repeated at the first scan position with an increase of the intensity and the frequency of the ultrasound, the focusing size, and the intensity of the light so as to obtain a light signal lac greater than the level of the shot noise $\alpha$ of the light sensor 7 in the light detecting unit 400. On the other hand, when a second focusing position different form the first focusing potion is set in S15, the ultrasonic intensity, the focusing size, which were once increased at the first focusing position, are reset to the initial value so as to retry a measurement.

This embodiment fixes a type of parameter which increases in step S16. In other words, only the focusing size is increased over the permissible number of times t when the focusing size is selected in S16. However, a different parameter may be selected the permissible number of times. For example, the focusing size is increased at the first time, and the ultrasound is increased at the second time.

This embodiment utilized the spectroscopic analysis method that uses the absorption spectrum characteristic of oxygenated hemoglobin and reduced hemoglobin, and a wavelength range between 600 and 1500 nm. However, the present invention is not limited to this embodiment but may use a main ingredient of a biological tissue, such as water, lipid, and protein (collagen), as an object of the spectroscopic analysis.

The measurement apparatus according to this embodiment can improve a SN ratio and obtain a measurement result by the slight sacrifice of the resolution when the SN ratio of the light signal of the modulated light is bad.

SECOND EMBODIMENT

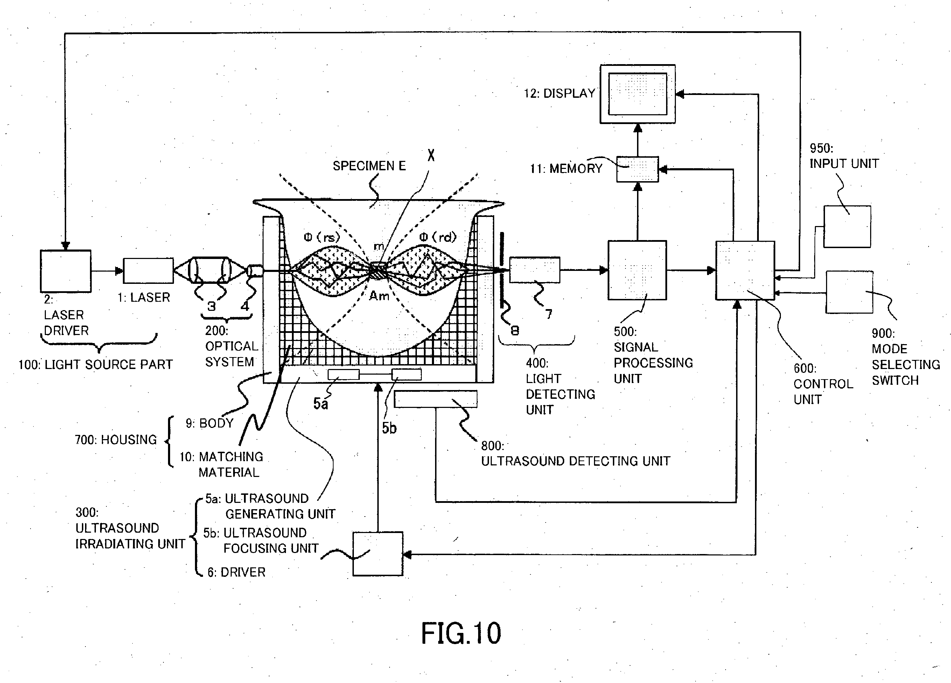

FIG. 10 is a block diagram of a measurement apparatus according to a second embodiment of the present invention. The measurement apparatus in this embodiment is similar to the measurement apparatus in the first embodiment, but differs in having a mode-selecting switch 900 and an input unit 950.

The mode-selecting switch 900 serves to switch the operation mode of the measurement apparatus between a rough scan mode and fine scan mode. The mode-selecting switch 900 may be integrated with the input unit 950.

The input unit 950 includes a keyboard, amouse, and another pointing device. The input unit 950 serves as an input unit used for an operator to input and set an ultrasonic focusing size and a measurement area as a scan area of the measurement site X in the specimen E. Hence, the input unit 950 serves as both a measurement area setting device and a focusing size setting device.

The first embodiment changes or increases the ultrasonic intensity, the ultrasonic focusing size in order to avoid a non-measurable state when the SN ratio of the light signal lac of the modulated light is equal to or smaller than the threshold value. On the other hand, it is unlikely that the entire specimen is abnormal. With the foregoing facts in mind, in measuring the entire specimen, this embodiment emphasizes a reduction of the measurement time period rather than an improvement of the resolution and emphasizes the improvement of the resolution rather than the reduction of the measurement time period for an abnormal site. Accordingly, this embodiment initially measures the entire specimen in the rough scan mode, and next when it detects an abnormal site in the rough scanning mode it measures the abnormal site in the fine scan mode. Of course, it may promptly start fine scanning without performing rough scanning previously when an abnormal site is already detected during previous measurements. However, in this embodiment, the apparatus performs fine scanning after rough scanning, because the specimen E is a breast having a structure that is likely to change and an abnormal site is likely to change. An abnormal site will be easily identified by performing rough scanning and fine scanning continuously.

The control unit 600 sets the focusing size of pulse of the ultrasound to be smaller at fine-scan mode than rough scan mode while the mode selecting switch 900 sets fine-scan mode. It is known that a smaller focusing size leads to a higher resolution.

The control unit 600 may automatically set the focusing size in the rough scan mode and the focusing size in the fine scan mode, or alternatively may prompt an operator to input the focusing size on the display 12. In the latter case, the control unit 600 may display candidates of the focusing sizes so that the operator can select one of the candidate sizes to input. If the operator manually inputs the size, the operator inputs and sets the focusing size via the input unit 950. The focusing size in the rough scan mode of this embodiment is approximately 10mm at which a tumor of a breast cancer is said to grow drastically. The focusing size in the fine scan mode of this embodiment is approximately several millimeters.

The control unit 600 may automatically set the measurement site which is determined abnormal in the rough scan mode, to an object of the fine scanning or may prompt an operator to input a set range for fine scanning as in this embodiment. In the latter case, the control unit 600 may display candidates in the measurable range to be input so that the operator can select one of them. When the operator manually inputs the candidate, the operator inputs and sets the measurable range via the input unit 950.

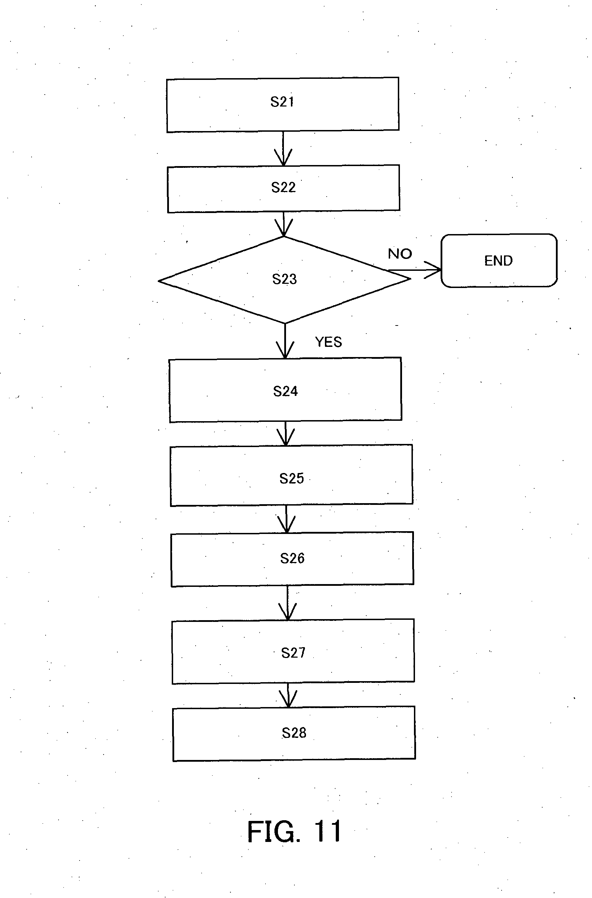

Referring now to FIG. 11, a description will be given of an operation of the measurement apparatus. FIG. 11 is a flowchart which describes how the measurement apparatus of this embodiment operates. First, an operator sets the mode-selecting switch 900 to the rough scan mode in step S21. Next, the control unit 600 automatically sets the ultrasound focusing size to D1, and implements rough scanning in step S22. The rough scanning is similar to that described in FIG. 9, and a description thereof will be omitted.

Next, the control unit 600 determines whether there is an abnormal site based on the results of S10 and S13 in step S23. If no abnormal sites are detected (when S23 answers NO), the flow is completed. If an abnormal site is detected (when S23 answers YES), the control unit 600 displays the existence and position of the abnormal site on the display 12 and stores them in the memory 11 in step S24. A final determination is left to an operator (or a doctor), since the abnormal site is a measurement site that is or can be abnormal. Even if the control unit 600 displays an abnormal site on the display 12 at S24, the operator may determines that it is normal and complete the measurement by his own decision. The operator switches the mode-selecting switch 900 to the fine scan mode to perform if a further measurement is necessary in step S25. In the fine scan mode, the operator sets the focusing size to D2 (< D1) via the input unit 950 in step S26, and sets the measurement area in the specimen E via the input unit 950 in step S27. The fine scan is implemented in this condition in step S28. The fine scanning is similar to the rough scanning or those described in FIG. 9 except that the focusing size is changed, and a description thereof will be omitted.

The measurement apparatus of this embodiment measures the entire specimen in rough scanning to shorten the measurement time period, and measures an abnormal site in fine scanning to improve the reliability of the diagnosis with a high resolution. In this way, the present invention can flexibly meet a measurement demand.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

## Claims

1. A measurement apparatus configured to measure a spectroscopic characteristic of a specimen by acousto-optical tomography, the measurement apparatus comprising:

    a light source (100) configured to provide light to be irradiated onto the specimen;
    an ultrasound source (5a) configured to generate ultrasound;
    ultrasound focusing means (5b) configured to focus the ultrasound generated by the ultrasound source on a measurement area of the specimen;
    light detecting means (400) configured to detect light modulated by the acousto-optical effect on the measurement area of the specimen;

    control means (600) configured to set initial values (D1, F1, Ω1) of parameters including an intensity of the ultrasound generated by the ultrasound source, a frequency (Ω1) of the ultrasound generated by the ultrasound source, an ultrasound focusing size (D1, D2) made by the ultrasound focusing means, **characterized in that**
    the control means (600) is further configured to change the initial value of at least one of the parameters based on an output of the light detecting means;
    and **in that** the apparatus further comprises signal analyzing means (500) configured to compare a signal level of the modulated light with a threshold value defined by a noise level of the light detecting means, and to communicate a comparison result to the control means,
    wherein when the signal level of the modulated light is equal to or smaller than the threshold value, the control means increases the initial value of at least one of the parameters.

2. A measurement apparatus according to claim 1, wherein the signal analyzing means generates coordinate data of a focusing position (X) and distribution data of a spectroscopic characteristic in the specimen from a light signal of the modulated light.

3. A measurement apparatus according to claim 1, wherein the control means is configured to scan a focusing position (D1) of the ultrasound focused by the ultrasound focusing means, and repeats the measurement at a first scanning position by increasing at least one of the intensity and frequency of the ultrasound, the focusing size of the ultrasound from the initial value when the signal to noise ratio of the modulated light is equal to or smaller than the threshold value at a first focusing position of the ultrasound, and provides a measurement at a second focusing position (D2) different from the first focusing position by resetting the at least one controlled parameter (s) to the initial value which has been increased for the first focusing position.

4. A measurement apparatus according to claim 1, further comprising:

    mode selecting means (900) adapted to allow a user to select either a rough scan mode or a fine scan mode,
    wherein the control means configured to set the ultrasound focusing size to be smaller in the fine scan mode than in the rough scan mode.

5. A measurement apparatus according to claim 4, further comprising measurement area setting means (950) configured to set a measurement area of a measurement site in the specimen.

6. A measurement apparatus according to claim 4, further comprising focusing size setting means (950) configured to set an ultrasound focusing position made by the ultrasound focusing means.

7. A measurement apparatus according to any one of claims 1 to 6, further comprising an ultrasound detectingmeans (5a) which detects the ultrasound, wherein control means controls the intensity of the ultrasound generated by the ultrasound source based on a detection result of the ultrasound detecting means.

8. A measurement apparatus according to claim 7, wherein the ultrasound source and the ultrasound detecting means constitutes a single ultrasound transducer (13).

9. A method of measuring a spectroscopic characteristic of a specimen by acousto-optical tomography, the method comprising:

setting initial values (D1, F1, $\Omega$1) of parameters by a control means (600), the parameters including an intensity of ultrasound generated by an ultrasound source (5a), a frequency ($\Omega$1) of the ultrasound generated by the ultrasound source, an ultrasound focusing size (D1, D2) made by an ultrasound focusing means (5b) ;
irradiating the specimen with light generated by the light source;
focusing ultrasound on a measurement area of the specimen by an ultrasound focusing means;
detecting light modulated by the acousto-optical effect on the measurement area of the specimen by a light detecting means (400);
comparing a signal level of the modulated light with a threshold value defined by a noise level of the light detecting means;
communicating a comparison result to the control means; **characterised in that** the method further comprises
changing the initial value of at least one of the parameters by the control means based on the modulated light detected by the light detecting means,
wherein when the signal level of the modulated light is equal to or smaller than the threshold value, the control means increases the initial value of at least one of the parameters.

**Patentansprüche**

1. Messvorrichtung, konfiguriert, um eine spektroskopische Charakteristik einer Probe durch akustopti-

sche Tomographie zu messen, wobei die Messvorrichtung umfasst:

eine Lichtquelle (100), konfiguriert, um Licht, mit dem die Probe zu bestrahlen ist, bereitzustellen;
eine Ultraschallquelle (5a), konfiguriert, um Ultraschall zu erzeugen;
eine Ultraschallfokussiereinrichtung (5b), konfiguriert, um den von der Ultraschallquelle erzeugten Ultraschall auf eine Messregion der Probe zu fokussieren;
eine Lichtdetektiereinrichtung (400), konfiguriert, um durch den akustooptischen Effekt an der Messregion der Probe moduliertes Licht zu detektieren;
eine Steuereinrichtung (600), konfiguriert, um Startwerte (D1, F1, $\Omega$1) von Parametern einschließlich einer Intensität des von der Ultraschallquelle erzeugten Ultraschalls, einer Frequenz ($\Omega$1) des von der Ultraschallquelle erzeugten Ultraschalls und einer von der Ultraschallfokussiereinrichtung geschaffenen Ultraschallfokussiergröße (D1, D2) einzustellen;
**dadurch gekennzeichnet, dass**
die Steuereinrichtung (600) weiterhin konfiguriert ist, um den Startwert von zumindest einem der Parameter basierend auf einer Ausgabe der Lichtdetektiereinrichtung zu ändern; und
dadurch, dass die Vorrichtung weiterhin umfasst:

eine Signalauswertungseinrichtung (500), konfiguriert, um ein Signalniveau des modulierten Lichts mit einem durch ein Rauschniveau der Lichtdetektiereinrichtung definierten Schwellenwert zu vergleichen und ein Vergleichsergebnis an die Steuereinrichtung zu übermitteln,
wobei, wenn das Signalniveau des modulierten Lichts gleich oder kleiner dem Schwellenwert ist, die Steuereinrichtung den Startwert von zumindest einem der Parameter erhöht.

2. Messvorrichtung nach Anspruch 1, wobei die Signalauswertungseinrichtung Koordinatendaten einer Fokussierposition (X) und Verteilungsdaten einer spektroskopischen Charakteristik in der Probe aus einem Lichtsignal des modulierten Lichts erzeugt.

3. Messvorrichtung nach Anspruch 1, wobei die Steuereinrichtung konfiguriert ist, um eine Fokussierposition (D1) des von der Ultraschallfokussiereinrichtung fokussierten Ultraschalls abzutasten, die Messung an einer ersten Abtastposition unter Erhöhen zumindest der Intensität und der Frequenz des Ultraschalls und/oder der Fokussiergröße des Ultraschalls von den Startwerten zu wiederholen, wenn das Signal-Rausch-Verhältnis des modulierten Lichts gleich oder kleiner dem Schwellenwert an der ersten Fokussierposition des Ultraschalls ist, und eine Messung an einer von der ersten Fokussierposition verschiedenen, zweiten Fokussierposition (D2) unter Zurücksetzen des zumindest einen gesteuerten Parameters auf den Startwert bereitzustellen, der für die erste Fokussierposition erhöht worden ist.

4. Messvorrichtung nach Anspruch 1, weiterhin umfassend:

eine Modusauswahleinrichtung (900), ausgebildet, um einem Benutzer zu ermöglichen, entweder einen Grobabtastmodus oder einen Feinabtastmodus auszuwählen, wobei die Steuereinrichtung konfiguriert ist, um die Ultraschallfokussiergröße im Feinabtastmodus kleiner als im Grobabtastmodus einzustellen.

5. Messvorrichtung nach Anspruch 4, weiterhin umfassend eine Messregionseinstelleinrichtung (950), konfiguriert, um eine Messregion eines Messgebiets in der Probe einzustellen.

6. Messvorrichtung nach Anspruch 4, weiterhin umfassend eine Fokussiergrößeneinstelleinrichtung (950), konfiguriert, um eine von der Ultraschallfokussiereinrichtung geschaffene Fokussierposition einzustellen.

7. Messvorrichtung nach einem der Ansprüche 1 bis 6, weiterhin umfassend eine Ultraschalldetektiereinrichtung (5a), die den Ultraschall detektiert, wobei die Steuereinrichtung die Intensität des von der Ultraschallquelle erzeugten Ultraschalls basierend auf dem Detektierergebnis der Ultraschalldetektiereinrichtung steuert.

8. Messvorrichtung nach Anspruch 7, wobei die Ultraschallquelle und die Ultraschalldetektiereinrichtung einen einzigen Ultraschallumwandler (13) bilden.

9. Verfahren zur Messung einer spektroskopischen Charakteristik einer Probe durch akustooptische Tomographie, wobei das Verfahren umfasst:

Einstellen von Startwerten (D1, F1, Ω1) von Parametern durch eine Steuereinrichtung (600), wobei die Parameter eine Intensität von durch eine Ultraschallquelle (5a) erzeugten Ultraschall, eine Frequenz (Ω1) des von der Ultraschallquelle erzeugten Ultraschalls und eine von einer Ultraschallfokussiereinrichtung (5b) geschaffene Ultraschallfokussiergröße (D1, D2) einschließen; Bestrahlen der Probe mit von einer Lichtquelle erzeugtem Licht; Fokussieren von Ultraschall auf eine Messregion der Probe durch eine Ultraschallfokussiereinrichtung; Detektieren von durch den akustooptischen Effekt an der Messregion der Probe moduliertem Licht durch eine Lichtdetektiereinrichtung (400); Vergleichen eines Signalniveaus des modulierten Lichts mit einem durch ein Rauschniveau der Lichtdetektiereinrichtung definierten Schwellenwert; und Übermitteln eines Vergleichsergebnisses an die Steuereinrichtung; weiterhin **gekennzeichnet durch**:

Ändern des Startwertes von zumindest einem der Parameter **durch** die Steuereinrichtung basierend auf dem von der Lichtdetektiereinrichtung detektierten modulierten Licht, wobei die Steuereinrichtung den Startwert von zumindest einem der Parameter erhöht, wenn das Signalniveau des modulierten Lichts gleich oder kleiner dem Schwellenwert ist.

**Revendications**

1. Appareil de mesure configuré pour mesurer une caractéristique spectroscopique d'un échantillon par tomographie acousto-optique, l'appareil de mesure comprenant :

une source de lumière (100) configurée pour fournir une lumière devant être projetée sur l'échantillon ; une source d'ultrasons (5a) configurée pour générer des ultrasons ; un moyen de focalisation des ultrasons (5b) configuré pour focaliser les ultrasons générés par la source d'ultrasons sur une zone de mesure de l'échantillon ; un moyen de détection de lumière (400) configuré pour détecter une lumière modulée par l'effet acousto-optique sur la zone de mesure de l'échantillon ; un moyen de commande (600) configuré pour régler des valeurs initiales (D1, F1, Ω1) de paramètres comprenant une intensité des ultrasons générés par la source d'ultrasons, une fréquence (Ω1) des ultrasons générés par la source d'ultrasons, une taille de focalisation des ultrasons (D1, D2) produite par le moyen de focalisation des ultrasons, **caractérisé en ce que** :

le moyen de commande (600) est en outre configuré pour modifier la valeur initiale d'au moins l'un des paramètres sur la base d'une sortie du moyen de détection de lumière ; et **en ce que** l'appareil comprend en outre un moyen d'analyse de signal (500) configuré pour comparer un niveau de signal de la lumière modulée à une valeur de seuil définie par un niveau de bruit du moyen de détection de lumière, et pour transmettre un résultat de comparaison au moyen de commande,

dans lequel, lorsque le niveau de signal de la lumière modulée est inférieur ou égal à la valeur de seuil, le moyen de commande augmente la valeur initiale d'au moins l'un des paramètres.

**2.** Appareil de mesure selon la revendication 1, dans lequel le moyen d'analyse de signal génère des données de coordonnées d'une position de focalisation (X) et des données de distribution d'une caractéristique spectroscopique dans l'échantillon à partir d'un signal de lumière de la lumière modulée.

**3.** Appareil de mesure selon la revendication 1, dans lequel le moyen de commande est configuré pour balayer une position de focalisation (D1) des ultrasons focalisés par le moyen de focalisation des ultrasons, et répète la mesure à une première position de balayage en augmentant au moins l'une de l'intensité et de la fréquence des ultrasons, la taille de focalisation des ultrasons par rapport à la valeur initiale lorsque le rapport signal à bruit de la lumière modulée est inférieur ou égal à la valeur de seuil à une première position de focalisation des ultrasons, et fournit une mesure à une seconde position de focalisation (D2) différente de la première position de focalisation en réinitialisant l'au moins un paramètre commandé à la valeur initiale qui a été augmentée pour la première position de focalisation.

**4.** Appareil de mesure selon la revendication 1, comprenant en outre :

un moyen de sélection de mode (900) apte à permettre à un utilisateur de sélectionner soit un mode de balayage grossier, soit un mode de balayage fin,

dans lequel le moyen de commande est configuré pour régler la taille de focalisation des ultrasons afin qu'elle soit inférieure dans le mode de balayage fin à celle du mode de balayage grossier.

**5.** Appareil de mesure selon la revendication 4, comprenant en outre un moyen de réglage de zone de mesure (950) configuré pour régler une zone de mesure d'un site de mesure dans l'échantillon.

**6.** Appareil de mesure selon la revendication 4, comprenant en outre un moyen de réglage de taille de focalisation (950) configuré pour régler une position de focalisation des ultrasons obtenue par le moyen de focalisation des ultrasons.

**7.** Appareil de mesure selon l'une quelconque des revendications 1 à 6, comprenant en outre un moyen de détection d'ultrasons (5a) qui détecte les ultrasons, dans lequel le moyen de commande commande l'intensité des ultrasons générés par la source d'ultrasons sur la base d'un résultat de détection du moyen de détection d'ultrasons.

**8.** Appareil de mesure selon la revendication 7, dans lequel la source d'ultrasons et le moyen de détection d'ultrasons constituent un transducteur à ultrasons unique (13).

**9.** Procédé de mesure d'une caractéristique spectroscopique d'un échantillon par tomographie acousto-optique, le procédé consistant à :

régler des valeurs initiales (D1, F1, $\Omega$1) de paramètres à l'aide d'un moyen de commande (600), les paramètres comportant une intensité d'ultrasons générés par une source d'ultrasons (5a), une fréquence ($\Omega$1) des ultrasons générés par la source d'ultrasons, une taille de focalisation des ultrasons (D1, D2) obtenue par un moyen de focalisation des ultrasons (5b) ;

exposer l'échantillon à de la lumière générée par la source de lumière ;

focaliser des ultrasons sur une zone de mesure de l'échantillon à l'aide d'un moyen de focalisation des ultrasons ;

détecter la lumière modulée par l'effet acousto-optique sur la zone de mesure de l'échantillon à l'aide d'un moyen de détection de lumière (400) ;

comparer un niveau de signal de la lumière modulée à une valeur de seuil définie par un niveau de bruit du moyen de détection de lumière ;

transmettre un résultat de comparaison au moyen de commande ; **caractérisé en ce que** le procédé consiste en outre à :

modifier la valeur initiale d'au moins l'un des paramètres à l'aide du moyen de commande sur la base de la lumière modulée détectée par le moyen de détection de lumière, dans lequel, lorsque le niveau de signal de la lumière modulée est inférieur ou égal à la valeur de seuil, le moyen de commande augmente la valeur initiale d'au moins l'un des paramètres.

FIG.1

FIG.2

z

x

13a    13c    13e    13g    13i    13k    13m

13b  | 13d |  13f |  13h|  13j |  13l

15

14

16b |  16d |  16f |  16h |  16j |  16l

16a    16c    16e    16g    16i    16k    16m

FIG.3

FIG.4

FIG.5

EP 2 036 490 B1

FIG.6

FIG.7

FIG.8

FIG. 9

FIG.10

```
        ┌─────────────────────┐
        │         S21         │
        └─────────────────────┘
                   │
                   ▼
        ┌─────────────────────┐
        │         S22         │
        └─────────────────────┘
                   │
                   ▼
              ╱─────────╲          NO      ┌──────────────┐
             ╱           ╲ ─────────────▶  │     END      │
             ╲    S23    ╱                 └──────────────┘
              ╲─────────╱
                   │      YES
                   ▼
        ┌─────────────────────┐
        │         S24         │
        └─────────────────────┘
                   │
                   ▼
        ┌─────────────────────┐
        │         S25         │
        └─────────────────────┘
                   │
                   ▼
        ┌─────────────────────┐
        │         S26         │
        └─────────────────────┘
                   │
                   ▼
        ┌─────────────────────┐
        │         S27         │
        └─────────────────────┘
                   │
                   ▼
        ┌─────────────────────┐
        │         S28         │
        └─────────────────────┘
```

FIG. 11

FIG.12

**EP 2 036 490 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6738653 B

- US 6002958 A

### Non-patent literature cited in the description

- **LIHONG V. WANG.** Ultrasonic Modulation of Scattered Light in Turbid Media and a Potential Novel Tomography in Biomedicine. *Photochemistry and Photobiology,* 1998, vol. 67 (1), 41-49
- **LIHONG V. WANG.** Mechanism of Ultrasonic modulation of Multiply Scattered Coherent Light: An Analytical Model. *Phys. Rev. Lett.,* 2001, vol. 87 (4

- **LIHONG V. WANG.** Ultrasonic modulation of multiply scattered coherent light: An analytical model for anistropically scattering media. *PHYSICAL REVIEW E,* 2002, vol. 66, 026603